# FASCICULE DE BREVET EUROPEEN

(11) **EP 3 848 446 B1**
(45) Date de publication et mention de la délivrance du brevet: **12.07.2023**
(21) Numéro de dépôt: 21150310.7
(22) Date de dépôt: 05.01.2021
(51) Int. Cl.: C12M 1/107, B01D 53/52, B01J 12/00, B01J 19/26, C10L 3/10, C12M 1/00

(54) **DIGESTEUR COMPRENANT UN SYSTEME D'INJECTION D'OXYGENE PRESENTANT UN MOYEN TUBULAIRE CONFORME EN QUADRILLAGE**
FAULBEHÄLTER MIT EINEM SAUERSTOFFINJEKTIONSSYSTEM, DAS EIN KONFORMES ROHRFÖRMIGES MITTEL IN GITTERFORM AUFWEIST
DIGESTER COMPRISING AN OXYGEN INJECTION SYSTEM HAVING A TUBULAR MEANS ARRANGED IN GRID PATTERN

(30) Priorité: 09.01.2020 FR 2000178
(43) Date de publication de la demande: 14.07.2021
(73) Titulaire: L'Air Liquide, société anonyme pour l'Étude et l'Exploitation des procédés Georges Claude, 75007 Paris (FR)
(72) Inventeur: BERTRANDIAS, Aude, 78350 Les Loges-En-Josas (FR); FRIMAT, David, 78350 Les Loges-En-Josas (FR); TRUEBA, Antonio, 78350 Les Loges-En-Josas (FR); SEIWERT, Jacopo, 78350 Les Loges-En-Josas (FR); OLLIER, Jérémy, 38360 Sassenage (FR); VALENTIN, Solène, 38360 Sassenage (FR)
(74) Mandataire: Air Liquide

(56) Documents cités:
- CN-U- 202 942 808
- US-A- 4 580 597
- US-A1- 2008 227 081
- US-A1- 2012 068 111

## Description

La présente invention est relative à une installation et un procédé pour la production de biogaz au moins partiellement désulfuré.

Le biogaz est le gaz produit lors de la dégradation de matières organiques en l'absence d'oxygène (digestion anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, et dont les conditions sont contrôlées, appelé méthaniseur ou digesteur, puis dans un post-digesteur, similaire au digesteur et permettant de pousser plus loin la réaction de méthanisation.

On appellera biomasse tout groupement de matières organiques pouvant se transformer en énergie à travers ce processus de méthanisation, par exemple les boues de station d'épuration, fumiers/lisiers, résidus agricoles, déchets alimentaires...

Le digesteur, c'est-à-dire le réacteur dédié à la méthanisation de la biomasse, est une cuve fermée, chauffée ou non (opération à une température fixée, entre la température ambiante et 55°C) et dont le contenu constitué de la biomasse est brassé, en continu ou séquentiel. Les conditions dans le digesteur sont anaérobies et le biogaz généré se retrouve dans l'espace de tête du digesteur (ciel gazeux), où il est prélevé. Les post-digesteurs sont similaires aux digesteurs.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH₄) et du dioxyde de carbone (CO₂) dans des proportions variables en fonction du mode d'obtention et du substrat mais peut également contenir, en moindres proportions de l'eau, de l'azote, de l'hydrogène sulfuré (H₂S), de l'oxygène, ainsi que des composés organiques autres, à l'état de traces, dont le H2S, entre 10 et 50,000 ppmv.

Selon les matières organiques dégradées et les techniques utilisées, les proportions des composants diffèrent, mais en moyenne le biogaz comporte, sur gaz sec, de 30 à 75% de méthane, de 15 à 60% de CO₂, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au coeur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (bioGNL)...

Selon la composition de la biomasse, le biogaz produit lors de la digestion contient du sulfure d'hydrogène (H₂S) dans des teneurs comprises entre 10 et 50 000 ppm.

Quelle que soit la destination finale de valorisation du biogaz, il s'avère indispensable d'éliminer le sulfure d'hydrogène qui est une impureté toxique et corrosive. De plus, si l'utilisation du biogaz consiste à l'épurer pour injecter du biométhane dans le réseau de gaz naturel, des spécifications strictes limitent la quantité de H₂S autorisée.

Plusieurs méthodes d'élimination du H₂S existent et sont plus ou moins répandues (lits de charbon actif, ajout de composés de fer, absorption physique ou chimique, lavages à l'eau, biofiltres...). L'élimination se fait principalement par adsorption sur lit de charbon actif, à l'extérieur du digesteur. Dans un nombre croissant de digesteurs, l'abattement de l'H2S se fait aussi en partie par injection d'air/air enrichi/O₂ dans le ciel gazeux du digesteur, ce qui constitue une solution in situ. Avec une injection dans le ciel gazeux à faible dose, du soufre solide est formé à partir du H₂S et O₂ (eq. (1)), réalisée par des bactéries sulfo-oxydantes e.g. Thiobacillus. A haute dose d'O₂ injecté, le milieu est acidifié (eq. (2)). La réaction (1) est donc ciblée.

H₂S + 0.5 O₂ → S + H₂O (1)

H₂S + 2 O₂ → SO₄ ²⁻ + 2 H + (2)

Les quantités d'injection d'O₂ nécessaires en pratiques sont différentes de celles attendues par la stoechiométrie de l'éq. (1): des doses de 0.3 - 3% d'O₂ par rapport au biogaz généré sont le plus souvent recommandées, avec des doses jusqu'à 12% qui sont parfois évoquées. Aujourd'hui, l'injection d'air/air enrichi/O₂ in situ n'est pas optimisée et les lits de charbon actif doivent donc être maintenus pour éliminer la totalité du H₂S.

Les solutions existantes consistent à injecter l'air/O2 dans le digesteur avec un seul point d'injection menant potentiellement à une réaction localisée (élimination de sulfure d'hydrogène localisée). En cas de dosage d'oxygène inadéquat, cela peut de plus mener à une accumulation locale d'oxygène non consommé, ce qui est indésirable car (i) le mélange de biogaz et d'oxygène est explosif au delà d'une certaine limite (ii) l'épuration du biogaz qui contient de l'oxygène est plus complexe (iii) l'oxygène accumulé n'est pas utilisé afin d'abattre le plus possible d'H2S et (iv) des zones aérobies peuvent être créées localement et inhibées la réaction de méthanisation.

De plus, le point unique d'injection est placé à l'opposé de la sortie du biogaz. Une part du biogaz peut donc directement être évacuée du digesteur, sans avoir pu réagir avec l'oxygène injecté. Il reste alors contaminé en sulfure d'hydrogène en sortie.

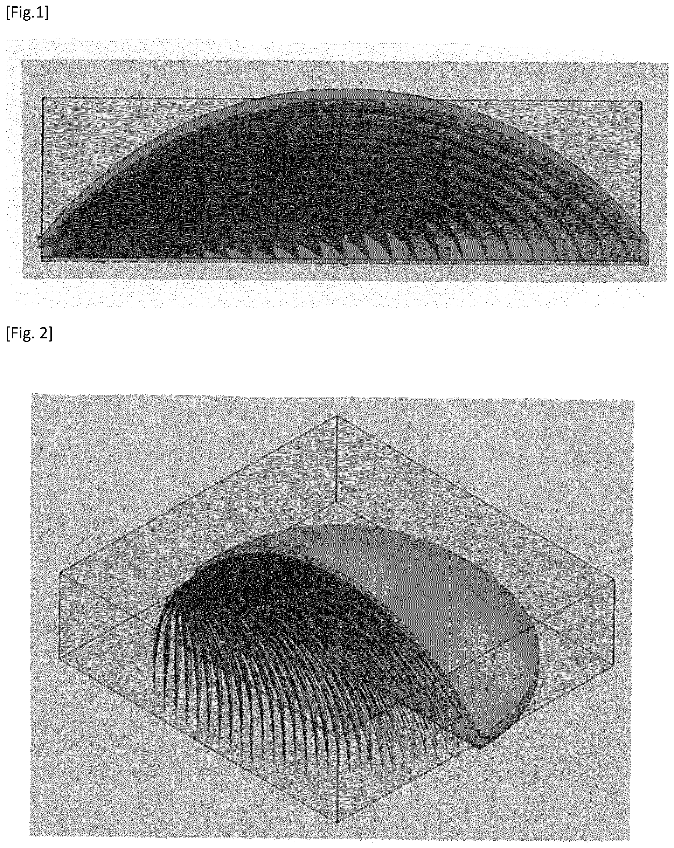

En effet, d'après des simulations sur un digesteur représentées aux figures 1 et 2 [Fig. 1] et [Fig. 2], on voit que le biogaz s'écoule en régime laminaire. Cela confirme que l'oxygène injecté en un point n'est pas brassé dans l'intégralité du ciel gazeux du digesteur, mais est emporté vers la sortie de gaz directement. Ainsi la réaction est localisée.

Ceci a été confirmé visuellement puisqu'il a été observé que le soufre solide se formait plutôt sur les surfaces du filet de désulfuration situées en périphérie et moins au centre. Le document US2008/227081 divulgue un réacteur de production de biogaz avec un système d'injection d'un gaz d'oxydation dans le ciel gazeux, comprenant un moyen tubulaire ayant un centre de symétrie dans l'axe de symétrie de l'enceinte du réacteur.

Partant de là, un problème qui se pose est de fournir une installation améliorée favorisant l'élimination plus poussée de l'H₂S.

Une solution de la présente invention est une installation de production de biogaz au moins partiellement désulfuré comprenant un digesteur et/ou un post-digesteur de biomasse, le digesteur et/ou le post-digesteur comprenant :
- Une enceinte comprenant la biomasse et le ciel gazeux, et
- Un système d'injection d'un gaz d'oxydation dans le ciel gazeux,
caractérisée en ce que le système d'injection comprend un moyen tubulaire conformé en quadrillage, ayant un centre de symétrie placé dans l'axe de symétrie de l'enceinte, et présentant des micro-injecteurs.

Par « ciel gazeux » on entend l'espace dans le digesteur ou post-digesteur contenant du gaz (par opposition à l'espace qui contient le liquide).

De préférence le quadrillage est formé par un ensemble de tubes.

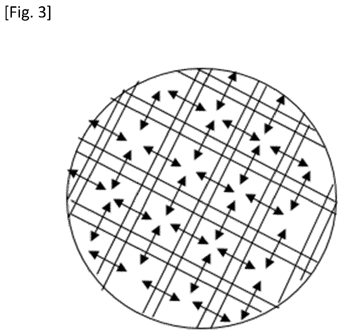

La figure 3 [Fig. 3] est une coupe schématique horizontale de l'enceinte du digesteur. Notons que la coupe est faite dans la portion de la paroi interne de l'enceinte située au niveau du ciel gazeux.

Selon le cas, l'installation selon l'invention peut présenter une ou plusieurs des caractéristiques ci-dessous :
- chaque carré du quadrillage comprend un micro-injecteur sur un de ses côtés.
- chaque carré du quadrillage comprend un micro-injecteur sur chacun de ses côtés.
- le quadrillage comprend au moins quatre carrés.
- au moins un micro-injecteur est situé à moins de deux mètres de l'axe de symétrie de l'enceinte.
- le moyen tubulaire est situé à moins d'un mètre de l'interface ciel gazeux - biomasse.
- l'installation comprend un filet de désulfurisation placé à l'horizontal et fixé dans la partie supérieure de l'enceinte et le moyen tubulaire est posé sur ledit filet.
- l'installation comprend un filet de désulfurisation placé à l'horizontal et fixé dans la partie supérieure de l'enceinte et le moyen tubulaire est suspendu à ce filet. Cette suspension se fait de préférence au moyen de cordes.
- le moyen tubulaire est composé d'un matériau résistant à une atmosphère humide et corrosive. Nous pouvons citer à titre d'exemple certains aciers inox, PEEK, PTFE.

L'installation selon l'invention permet de répartir les doses du gaz d'oxydation injectées dans l'ensemble du digesteur, de manière plus homogène. Ainsi les réactions ne seront plus localisées, permettant de mieux abattre la globalité du sulfure d'hydrogène.

De plus cette répartition plus homogène du gaz d'oxydation dans le ciel gazeux du digesteur permet une optimisation de la quantité d'oxygène utilisé, et de réduire sa consommation. Dans le cas d'injection de l'air ou de l'air enrichi, la quantité d'azote dans le biogaz sera donc minimale.

La présente invention a également pour objet un procédé de production de biogaz au moins partiellement désulfuré mettant en oeuvre une installation selon l'invention, comprenant :
- L'injection de biomasse dans le digesteur ;
- L'injection d'un gaz d'oxydation dans le ciel gazeux du digesteur via le système d'injection du gaz d'oxydation ; et
- Le brassage de la biomasse.

De préférence le débit d'injection du gaz d'oxydation sera compris entre 0,3 et 3% du volume du biogaz produit.

Notons que le gaz d'oxydation pourra être de l'oxygène ou de l'air ou de l'air enrichi. Par air enrichi on entend de l'air présentant une teneur en oxygène plus élevée que la teneur en oxygène habituellement comprise dans l'air.

La solution selon l'invention permet d'obtenir un flux de biogaz comprenant moins de 200 ppm de sulfure d'hydrogène.

L'invention permet de réduire le coût d'épuration du sulfure d'hydrogène du biogaz de façon efficace sans besoin complexe en ingénierie.

## Revendications

1. Installation de production de biogaz au moins partiellement désulfuré comprenant un digesteur et/ou un post-digesteur de biomasse, le digesteur et/ou le post-digesteur comprenant :
- Une enceinte comprenant la biomasse et le ciel gazeux, et
- Un système d'injection d'un gaz d'oxydation dans le ciel gazeux,
**caractérisée en ce que** le système d'injection comprend un moyen tubulaire conformé en quadrillage, ayant un centre de symétrie placé dans l'axe de symétrie de l'enceinte, et présentant des micro-injecteurs.

2. Installation selon la revendication 1, **caractérisée en ce que** chaque carré du quadrillage comprend un micro-injecteur sur un de ses côtés.

3. Installation selon l'une des revendications 1 ou 2, **caractérisée en ce que** chaque carré du quadrillage comprend un micro-injecteur sur chacun de ses côtés.

4. Installation selon l'une des revendications 1 à 3, **caractérisée en ce que** le quadrillage comprend au moins quatre carrés.

5. Installation selon l'une des revendications 1 à 4, **caractérisée en ce qu'**au moins un micro-injecteur est situé à moins de deux mètres de l'axe de symétrie de l'enceinte.

6. Installation selon l'une des revendications 1 à 5, **caractérisée en ce que** le moyen tubulaire est situé à moins d'un mètre de l'interface ciel gazeux - biomasse.

7. Installation selon l'une des revendications 1 à 6, **caractérisée en ce qu'**elle comprend un filet de désulfurisation placé à l'horizontal et fixé dans la partie supérieure de l'enceinte et le moyen tubulaire est posé sur ledit filet.

8. Installation selon l'une des revendications 1 à 6, **caractérisée en ce qu'**elle comprend un filet de désulfurisation placé à l'horizontal et fixé dans la partie supérieure de l'enceinte et le moyen tubulaire est suspendu à ce filet.

9. Installation selon l'une des revendications 1 à 8, **caractérisée en ce que** le moyen tubulaire est composé d'un matériau résistant à une atmosphère humide et corrosive.

10. Procédé de production de biogaz au moins partiellement désulfuré mettant en oeuvre une installation selon l'une des revendications 1 à 9, comprenant :
- L'injection de biomasse dans le digesteur ;
- L'injection d'un gaz d'oxydation dans le ciel gazeux du digesteur via le système d'injection du gaz d'oxydation ; et
- Le brassage de la biomasse.

11. Procédé selon la revendication 10, **caractérisé en ce que** le gaz d'oxydation est de l'oxygène ou de l'air ou de l'air enrichi.

## Patentansprüche

1. Anlage zur Erzeugung von wenigstens teilweise entschwefeltem Biogas, die ein Fermenter und/oder einen Nachfermenter für Biomasse umfasst, wobei der Fermenter und/oder der Nachfermenter Folgendes umfasst:
- Ein Gehäuse, das die Biomasse und den Gasraum umfasst, und
- Ein System zur Injektion eines Oxidationsgases in den Gasraum, **dadurch gekennzeichnet, dass** das Injektionssystem ein gitterartig ausgebildetes rohrförmiges Mittel umfasst, das ein Symmetriezentrum aufweist, das in der Symmetrieachse des Gehäuses platziert ist, und Mikroinjektoren aufweist.

2. Anlage nach Anspruch 1, **dadurch gekennzeichnet, dass** jedes Quadrat des Gitters einen Mikroinjektor auf einer seiner Seiten umfasst.

3. Anlage nach einem der Ansprüche 1 oder 2, **dadurch gekennzeichnet, dass** jedes Quadrat des Gitters einen Mikroinjektor auf jeder seiner Seiten umfasst.

4. Anlage nach einem der Ansprüche 1 bis 3, **dadurch gekennzeichnet, dass** das Gitter wenigstens vier Quadrate umfasst.

5. Anlage nach einem der Ansprüche 1 bis 4, **dadurch gekennzeichnet, dass** sich wenigstens ein Mikroinjektor weniger als zwei Meter von der Symmetrieachse des Gehäuses entfernt befindet.

6. Anlage nach einem der Ansprüche 1 bis 5, **dadurch gekennzeichnet, dass** sich das röhrenförmige Mittel weniger als einen Meter von der Gasraum-Biomasse-Grenzfläche entfernt befindet.

7. Anlage nach einem der Ansprüche 1 bis 6, **dadurch gekennzeichnet, dass** sie ein Entschwefelungsnetz umfasst, das horizontal platziert ist und im oberen Abschnitt des Gehäuses befestigt ist und das rohrförmige Mittel auf das Netz gelegt ist.

8. Anlage nach einem der Ansprüche 1 bis 6, **dadurch gekennzeichnet, dass** sie ein Entschwefelungsnetz umfasst, das horizontal platziert ist und im oberen Abschnitt des Gehäuses befestigt ist und das rohrförmige Mittel an dieses Netz gehängt ist.

9. Anlage nach einem der Ansprüche 1 bis 8, **dadurch gekennzeichnet, dass** das rohrförmige Mittel aus einem Material zusammengesetzt ist, das einer feuchten und ätzenden Atmosphäre widersteht.

10. Verfahren zur Erzeugung von wenigstens teilweise entschwefeltem Biogas unter Verwendung einer Anlage nach einem der Ansprüche 1 bis 9, umfassend:
- Das Injizieren von Biomasse in den Fermenter;
- Das Injizieren eines Oxidationsgases in den Gasraum des Fermenters über das System zur Injektion des Oxidationsgases; und
- Das Umrühren der Biomasse.

11. Verfahren nach Anspruch 10, **dadurch gekennzeichnet, dass** das Oxidationsgas Sauerstoff oder Luft oder angereicherte Luft ist.

## Claims

1. Plant for producing at least partially desulfurized biogas, comprising a biomass digester and/or post-digester, the digester and/or post-digester comprising:
- a chamber comprising the biomass and the gas space, and
- a system for injecting an oxidizing gas into the gas space,
**characterized in that** the injection system comprises a tubular means formed in a grid pattern, having a centre of symmetry placed in the axis of symmetry of the chamber, and having micro-injectors.

2. Plant according to Claim 1, **characterized in that** each square of the grid pattern comprises a micro-injector on one of its sides.

3. Plant according to either of Claims 1 and 2, **characterized in that** each square of the grid pattern comprises a micro-injector on each of its sides.

4. Plant according to one of Claims 1 to 3, **characterized in that** the grid pattern comprises at least four squares.

5. Plant according to one of Claims 1 to 4, **characterized in that** at least one micro-injector is located less than two metres from the axis of symmetry of the chamber.

6. Plant according to one of Claims 1 to 5, **characterized in that** the tubular means is located less than one metre from the gas space-biomass interface.

7. Plant according to one of Claims 1 to 6, **characterized in that** it comprises a desulfurization net placed horizontally and fastened in the upper portion of the chamber and the tubular means is placed on said net.

8. Plant according to one of Claims 1 to 6, **characterized in that** it comprises a desulfurization net placed horizontally and fastened in the upper portion of the chamber and the tubular means is suspended from this net.

9. Plant according to one of Claims 1 to 8, **characterized in that** the tubular means is composed of a material which is resistant to a humid and corrosive atmosphere.

10. Process for producing at least partially desulfurized biogas, using a plant according to one of Claims 1 to 9, comprising:
- injecting biomass into the digester;
- injecting an oxidizing gas into the gas space of the digester via the system for injecting oxidizing gas; and
- mixing the biomass.

11. Process according to Claim 10, **characterized in that** the oxidizing gas is oxygen or air or enriched air.
